# EUROPEAN PATENT APPLICATION

(11) **EP 1 595 506 A1**
(43) Date of publication of application: **16.11.2005**
(21) Application number: 04712700.6
(22) Date of filing: 19.02.2004
(51) Int. Cl.: A61B 17/12

(54) **INDWELLING INSTRUMENT FOR FORMING EMBOLUS**

(30) Priority: 20.02.2003 JP 2003042724
(71) Applicant: Kaneka Medix Corporation, Osaka-shi, Osaka 530-8288 (JP); MARUHO HATSUJYO KOGYO CO., LTD., Kyoto-shi, Kyoto (JP)
(72) Inventor: TAKEUCHI, Masahiro, c/o Maruho Hatsujo Kogyo K. K., Kameoka-shi, Kyoto 621-0015 (JP); NISHIDA, Takahiko, c/o Maruho Hatsujo Kogyo K. K., Kameoka-shi, Kyoto 621-0015 (JP); SAKAI, Shinichi, c/o Kaneka Medix Corporation, Ashigarakami-gun, Kanagawa 258-0113 (JP); OGAWA, Atsushi, c/o Kaneka Medix Corporation, Ashigarakami-gun, Kanagawa 258-0113 (JP)
(74) Representative: Grünecker, Kinkeldey, Stockmair & Schwanhäusser Anwaltssozietät
(86) International application number: PCT/JP2004/001864
(87) International publication number: WO 2004/073528

(57) **Abstract**

Disclosed herein is an intracorporeally indwelling device for embolization, which is capable of being placed surly in an appointed site, capable of permiting surely performing a replacing operation of the intracorporeally indwelling device arranged once, and thus has high safety and high operating ability.

The intracorporeally indwelling device for embolization of the invention comprises a flexible coiled body and an axial extension-controlling member having specified tensile break strength and provided in the interior of the coiled body so as to extend in an axial direction of the coiled body. The axial extension-controlling member is made up of a wire material having tensile break strength of at least 700 N/mm² in the axial direction of the coiled body and a diameter of at most 25 µm.

## Description

### TECHNICAL FIELD

The present invention relates to an intracorporeally indwelling device for embolization, and particularly to an intracorporeally indwelling device for embolization used in, for example, alteration or blocking of a blood stream and embolization of a lesion site.

### BACKGROUND ART

As a treatment method for aneurysm or the like, which causes little invasion, there is currently known vascular embolization in which an intracorporeally indwelling device for embolization is placed within aneurysm. In this vascular embolization, the intracorporeally indwelling device for embolization placed within the aneurysm serves as a physical obstacle to a blood stream and can reduce the risk of aneurysmal rupture by forming a thrombus around the intracorporeally indwelling device for embolization. Examples of the intracorporeally indwelling device for embolization to be placed at an appointed site in a blood vessel, such as aneurysm, include intracorporeally indwelling devices (hereinafter also referred to as "coil for embolization") for embolization composed of a metal coil (see, for example, Prior Art. 1, Prior Art. 2 and Prior Art. 3).

Such a coil for embolization is introduced into aneurysm through a proper catheter by a push-out means (guide) detachably connected to an end thereof and separated and placed at an appointed site to use it.

Thus, such a coil for embolization is required to have various properties such as properties described below.
(1) It is to have high flexibility required for conducting a placement operation without causing problems such as break through of the wall surface of an application site such as a blood vessel or aneurysm by, for example, applying an excessive load to the blood vessel or aneurysm.
   The coil for embolization is required to have the high flexibility, also from the viewpoint that it is capable of being easily pushed into a small gap at the application site upon the placement operation to increase a rate of embolization (a rate of occupation of the coil for embolization in the appointed site) per unit volume as much as possible to prevent occurance of re-perfusion (compaction) by a formation of a gap by a change in shape with time after performing, for example, vascular embolization.
(2) It is to have a function of preventing or inhibiting a coiled body from endlessly expanding so as to permit surely conducting a replacement operation that the coiled body is recovered to correct its position after the coiled body is pushed out of a catheter and arranged at an appointed site once. In other words, since there is a possibility that the coiled body may be extended due to, for example, being caught on a leading end of the catheter, thereby encountering difficulty on recovery of the coiled body or damaging the application site, the coil is required to be so constructed that such a problem is not caused.
   Prior Art. 1: Japanese Patent Registration No. 3023076;
   Prior Art. 2: Japanese Patent Registration No. 2909021;
   Prior Art. 3: Japanese Patent Application Laid-Open No. 187248/1996.

### DISCLOSURE OF THE INVENTION

The present invention has been made on the basis of the foregoing circumstances and has as its object the provision of an intracorporeally indwelling device for embolization, which is capable of being surely introduced and placed into an appointed site in a vital body, capable of permitting surely performing a replacement operation of the intracorporeally indwelling device that it is recovered to correct its position, for example, after it is arranged once even when the replacement operation of the intracorporeally indwelling device is conducted, and thus has high safety and high operating ability.

### EFFECTS OF THE INVENTION

According to the intracorporeally indwelling device for embolization of the present invention, a wire material making up an axial extension-controlling member is made up of a material having a specified diameter, whereby the wire material itself is formed in a state having high flexibility, so that the axial expansion-controlling member is deformed by following the expansion of a coiled body. Thus, the axial expansion-controlling member does not impair the flexibility of the coiled body, and the intracorporeally indwelling device can be made up as that with high flexibility. Accordingly, high operating ability can be achieved upon conducting a placement operation, and the indwelling device can be surely introduced and placed into an appointed site through a proper catheter.

In addition, the axial expansion-controlling member is arranged within the coiled body, and the axial expansion-controlling member itself is formed as that having high tensile break strength, whereby the extension of the coiled body in an axial direction thereof can be controlled. Accordingly, when the replacement operation that the intracorporeally indwelling device is recovered to correct its position, for example, after the device is arranged in a vital body once, the coiled body is pulled into a catheter in a state that the coiled body has been prevented by the axial extension-controlling member from being more expanded, so that the replacement operation including the recovery of the intracorporeally indwelling device can be surely performed, and the intracorporeally indwelling device can be made up as that with high safety.

### BRIEF DESCRIPTION OF THE DRAWINGS

Fig. 1 is a cross-sectional view schematically illustrating the construction of an intracorporeally indwelling device for embolization according to an embodiment of the present invention.
Fig. 2 is an explanatory view for explaining a method for determining the flexibility of the intracorporeally indwelling device in the present invention.
Fig. 3 is a cross-sectional view schematically illustrating the construction of an intracorporeally indwelling device for embolization according to another embodiment of the present invention.
Fig. 4 is a cross-sectional view schematically illustrating the construction of an intracorporeally indwelling device for embolization according to a further embodiment of the present invention.
Fig. 5 is a cross-sectional view schematically illustrating the construction of an intracorporeally indwelling device for embolization according to a still further embodiment of the present invention.

### [Description of characters]

- 10: Intracorporeally indwelling device for embolization
- 11: Metal coil
- 12: Head part
- 13: Holding member
- 20: Axial expansion-controlling member
- 21: Metal wire
- 21A: Twisted wire
- 25: Stopping member
- 30: Axial expansion-controlling member
- 31: Fiber element
- C: Fixed position

### BEST MODE FOR CARRYING OUT THE INVENTION

The embodiments of the present invention will hereinafter be described in detail.

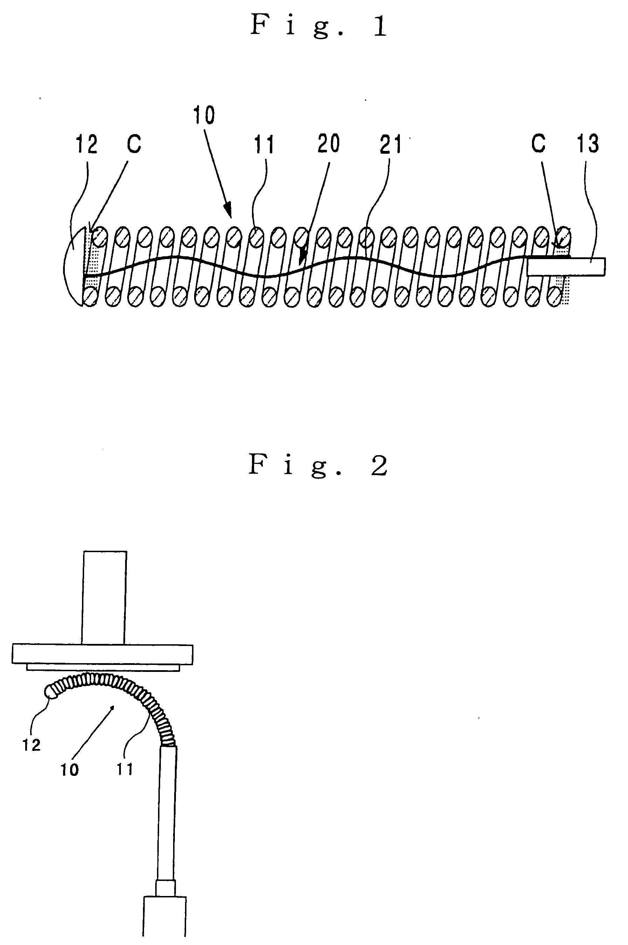

Fig. 1 is a cross-sectional view schematically illustrating the construction of an intracorporeally indwelling device for embolization according to an embodiment of the present invention.

This intracorporeally indwelling device for embolization (hereinafter referred to as "intracorporeally indwelling device" merely) 10 is equipped with a metal coil 11 making up a flexible coiled body, and a rounded head 12 is provided at a distal end of the metal coil 11. A holding member 13 in the form of, for example, a rod for holding the metal coil 11 is provided at a proximal end of the metal coil 11 so as to project and extend from the proximal end of the metal coil 11 to outside (the right-hand side in FIG. 1) in an axial direction of the coiled body in a state that a part thereof has been fixed to an inner peripheral surface at the proximal end of the metal coil 11.

The holding member 13 serves to hold the metal coil 11 in a state capable of being connected to and separated from a proper delivery wire, catheter or the like.

The metal coil 11 making up the intracorporeally indwelling device 10 is formed by spirally winding a metallic wire material. Such a metallic wire material may be selected from among those (biocompatible materials) that do not adversely affect a human body when placed within the human body for a long period of time. As examples thereof, may be mentioned tungsten, titanium, gold, platinum and alloys thereof, and stainless steel.

As already described above, the metal coil 11 making up the intracorporeally indwelling device 10 has flexibility or bendability and preferably has such a structure as described below though it varies according to a material of the metal wire material making up the metal coil 11.

For example, a diameter (element wire diameter) of the metallic wire material making up the metal coil 11 is 10 to 120 µm, a coil diameter of the metal coil 11 is 100 to 400 µm, a coil length is 2 to 500 mm, and the number of turns per unit length (1 mm) is 1 to 100.

In this intracorporeally indwelling device 10, an axial expansion-controlling member 20 composed of, for example, a metal wire 21 is arranged in the interior (within the tube hole) of the metal coil 11 in a state that one end thereof and the other end have been fixed to the distal end and proximal end of the metal coil 11, respectively, in such a manner that the whole thereof extends in the axial direction of the metal coil 11 in a state forming a spiral structure, thereby freely expandable within a controlled range. Portions (indicated by an arrow C) hatched by a dotted pattern in Fig. 1 indicate portions where the metal wire 21 is fixed.

The metal wire 21 making up the axial expansion-controlling member 20 is made up of a material at least having tensile break strength greater than minimum stress required for the metal coil 11 to be permanently deformed by the axial extension thereof in the axial direction of the metal coil, and actually made up of a metallic wire material having high tensile break strength inherent in the material in order to surely prevent the metal coil 11 from endlessly expanding in the axial direction thereof and moreover not to impair the flexibility of the metal coil 11 itself and having an element wire diameter as small as possible within limits that the expected strength of the axial expansion-controlling member 20 is surely retained.

Specifically, the metal wire 21 is made up of a metallic wire material that the tensile break strength inherent in the wire material making up the metal wire 21 is, for example, at least 700 N/mm², preferably 900 to 5,000 N/mm². As examples of such a metallic wire material, may be mentioned tungsten, titanium, gold, platinum and alloys thereof, and stainless steel. Among these, tungsten or a tungsten alloy is preferably used from the viewpoint of higher strength and more excellent working ability compared with other metal materials.

The metal wire 21 is made up of a wire material having a diameter (element wire diameter) of at most 25 µm, preferably 5 to 20 µm.

The wire material making up the metal wire 21 may be any of a round wire, a square wire, a flat wire and wire materials having any other sectional form than those described above.

The axial expansion-controlling member 20 in this intracorporeally indwelling device 10 is preferably such that the tensile break strength in the case where the construction such as the material and wire diameter of the metal wire 21 is specified is, for example, at least 0.1 N, more preferably 0.2 to 1 N. The term "tensile break strength [N]" as used herein means a value inherent to the axial expansion-controlling member specified in the construction such as, for example, the material and wire diameter of the metal wire 21 and indicated by a maximum load required for the axial expansion-controlling member to be broken. The tensile break strength of the whole of the axial expansion-controlling member 20 in the intracorporeally indwelling device 10 shown in Fig. 1 substantially corresponds to the tensile break strength inherent in the metallic wire material making up the metal wire 21.

The overall length of the metal wire 21 is preferably greater by 0.1 to 30.0%, more preferably 0.1 to 3.0% than the coil length of the metal coil 11 in an ordinary state.

No particular limitation is imposed on a fixing means between the distal end of the metal wire 21 and the head 12 and a fixing means between the proximal end of the metal wire 21 and the holding member 13. For example, adhesion with an adhesive, welding, bonding under pressure, physical connection and other means may be used.

In the intracorporeally indwelling device 10 according to the present invention, the flexibility of the whole of the intracorporeally indwelling device 10 is preferably at most 30 mN/mm, more preferably 1 to 25 mN/mm. In the present invention, "the flexibility" is indicated by a compressive modulus measured in the following manner.

There may be used, for example, a method in which a load is gradually applied to the intracorporeally indwelling device 10 in the axial direction thereof from above, as illustrated in Fig. 2, in a state that the intracorporeally indwelling device 10 has been fixed in such a manner that the coil axis of the metal coil 11 extends in, for example, a vertical direction (vertical direction in Fig. 2), and stress at the time the 1 pitch of the distal end of the metal wire 11 has been pressed and bent is measured as a compressive modulus.

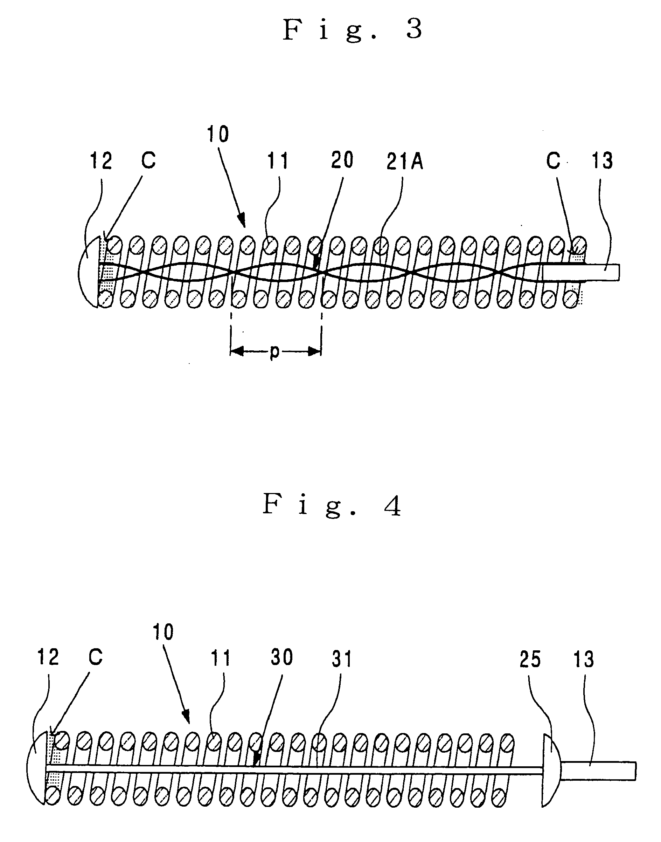

The axial expansion-controlling member 20 making up the intracorporeally indwelling device 10 according to the present invention may be made up of a twisted wire 21A formed by loosely twisting a plurality of metal wires as illustrated in Fig. 3. As materials for forming the respective metal wires, may be used those exemplified as the materials for forming the metal wire 21 in the intracorporeally indwelling device 10 shown in Fig. 1.

In this case, when the respective metal wires forming the twisted wire 21A are composed of the same metallic wire material as in the metal wire 21 in the intracorporeally indwelling device 10 shown in Fig. 1, the diameter (element wire diameter) thereof may be made smaller than that of the metal wire 21 in the intracorporeally indwelling device 10 shown in Fig. 1.

Specifically, the dimension of the diameter of each of the metal wires may be suitably set in such a manner that, for example, the tensile break strength of the whole of the twisted wire 21A is, for example, at least 0.1 N though it varies according to the number and material of metal wires to be twisted, or the like.

In the present invention, "a loosely twisted state" means a state that a pitch p of the twisted wire 21A, which is indicated by a length between 2 points where the respective metal wires intersect each other as illustrated in Fig. 3, is, for example, 0.05 to 3.0 mm, preferably 0.1 to 1.5 mm, or a state that tension (twisting stress) of, for example, 100 to 690 N/mm², preferably 150 to 600 N/mm² is applied when the metal wires are twisted to form the twisted wire.

The twisted wire 21A is formed by loosely twisting a plurality of the metal wires, whereby the axial expansion-controlling member 20 can be surely made up as that having high flexibility while it has the expected strength.

The axial expansion-controlling member 20 making up the intracorporeally indwelling device 10 according to the present invention may be made up of a fibrous polymer material. Examples of such a material include polyester resins such as polyethylene terephthalate (PET), polyolefin resins such as, for example, polypropylene (PP), and polyamide resins such as, for example, nylon.

As illustrated in Fig. 4, the intracorporeally indwelling device 10 according to the present invention may be so constructed that an axial expansion-controlling member 30 made up of a fiber element 31 composed of, for example, a resin is provided so as to project and extend from a proximal edge of a metal coil 11 outside in an axial direction of the coil by being not fixed to the metal coil 11 except that one end thereof is fixed to a head 12 in a state that the other end has no connection with the metal coil 11, and a stopping member 25 for prohibiting expansion of the metal coil 11 in the axial direction of the coil beyond a certain limit is provided at the other end of the fiber element 31.

The resin making up the fiber element 31 may be selected from among those that do not adversely affect a human body when placed within the human body for a long period of time. As specific examples thereof, may be mentioned vinyl polymers such as polyvinyl alcohol, polyvinyl chloride and polyvinyl acetate, olefin polymers such as ethylene-vinyl alcohol, polyethylene and polypropylene, polymers and copolymers such as nylon, polyester, polystyrene and polyurethane, and naturally derived polymers such as cellulose and chitin-chitosan.

The fiber element 31 making up the axial expansion-controlling member 30 is made up of that the diameter of which is the minimum size to the limit required to achieve expected tensile break strength in the axial direction of the metal coil 11. Specifically, it is made up of a wire material having a diameter of at most 25 µm, preferably 5 to 20 µm.

The fiber element 31 is preferably so constructed that a plurality of wire materials having a unit wire diameter (diameter of each wire material) of 5 to 15 um, preferably 5 to 10 µm are arranged so as to align in the axial direction of the coil.

The length of the fiber element 31 is preferably greater by 0.1 to 30.0%, more preferably 0.1 to 3.0% than the coil length of the metal coil 11 in an ordinary state, whereby a degree that the flexibility of the metal coil 11 is limited by the fiber element 31 can be surely made small which is below a certain limit.

No particular limitation is imposed on the form of the stopping member 25 so far as it has a size greater than the inner diameter of the metal coil 11 and does not damage an appointed site. In the embodiment illustrated, the stopping member is formed in, for example, a smooth semisphere having an outer diameter greater than the coil diameter of the metal coil 11.

The stopping member 25 is preferably provided at a position outside by, for example, 0.1 to 30.0% of the coil length from the proximal edge of the metal coil 11 in the ordinary state.

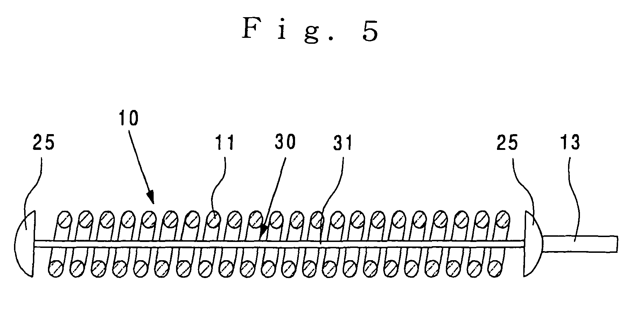

As illustrated in Fig. 5, the intracorporeally indwelling device 10 according to the present invention may be so constructed that a fiber element 31 making up an axial expansion-controlling member 30 is provided within a metal coil 11 so as to project and extend from both edges of the metal coil 11 outside in an axial direction of the coil in a state that it is not fixed to the metal coil 11 at all, and stopping members 25, 25 for prohibiting expansion of the metal coil 11 in the axial direction of the coil beyond a certain limit are respectively provided at both ends of the fiber element 31.

Each of the intracorporeally indwelling devices 10 described above may be used in a state that a linear form is held as it is. However, it is preferably used in a state that the coiled body composed of the linear metal coil 11 has been spirally wound to form a secondary coil, for example, in a state shaped to secondary form such as an "S-shaped" form, a "J-shaped" form, a three-dimensional structure or the like. When it is shaped to, for example, the secondary coil, the diameter of the secondary coil may be, for example, 1 to 45 mm.

A method for using the intracorporeally indwelling device 10 will hereinafter be described taking, as an example, a case where it is applied to treatment for aneurysm.

A proper catheter is first percutaneously inserted into a blood vessel with a pricking needle and arranged in such a manner that a leading end of the catheter reaches an aditus of aneurysm, the intracorporeally indwelling device 10 pre-shaped to a secondary form such as, for example, a secondary coil is detachably attached to a leading end of a guide wire, the guide wire is then inserted into the catheter and advanced in a state that the intracorporeally indwelling device 10 has been stretched in a straight line to return its form to a primary form (form illustrated in Figs. 1 to 4), and the intracorporeally indwelling device 10 is pushed out of the leading end of the catheter, thereby arranging it within the aneurysm.

In the present invention, as examples of a method for attaching the intracorporeally indwelling device 10 to the guide wire, may be mentioned (1) a method comprising taking a structure that the holding member 13 in the intracorporeally indwelling device 10 is mechanically joined to the guide wire and mechanically detached, and (2) a method comprising taking a structure that the holding member 13 in the intracorporeally indwelling device 10 is electrolytically separated by supplying, for example, a monopolar high-frequency current, thereby heating the holding member 13 by the high-frequency current to melt and sever it so as to separate from the guide wire.

When the intracorporeally indwelling device 10 is pushed out of the catheter, its form is restored to the form of the secondary coil to become a three-dimensionally entangled form. In this state, after the fact that the intracorporeally indwelling device 10 is completely inserted into the aneurysm is confirmed by fluoroscopy, the intracorporeally indwelling device 10 is separated from the leading end of the guide wire and placed.

A plurality of the intracorporeally indwelling devices 10 are used to conduct such an operation as described above repeatedly as needed, and the interior of the aneurysm is filled with a plurality of the intracorporeally indwelling devices 10 to form a thrombus, thereby inhibiting blood from flowing into the aneurysm. As a result, aneurysmal rupture can be surely prevented.

According to the intracorporeally indwelling devices 10 of the present invention, the intracorporeally indwelling devices 10 can be made up as that with high flexibility, whereby high operating ability can be achieved upon conducting a placement operation, and the indwelling devices can be surely introduced and placed into appointed sites through a proper catheter. In addition, the replacement operation including the recovery of the intracorporeally indwelling devices 10 can be surely performed, and the intracorporeally indwelling devices 10 can be made up as those having high safety.

More specifically, in the intracorporeally indwelling device (see Fig. 1) that both ends of the metal wire 21 making up the axial expansion-controlling member 20 are fixed to the metal coil 11, the axial expansion-controlling member 20 is made up of a wire material having tensile break strength greater than specified magnitude in the axial direction of the coil and a specified diameter of at most 25 µm, whereby the axial expansion-controlling member 20 itself is made up in a state having high flexibility, so that the metal wire 21 can be deformed following the expansion of the metal coil 11 in the axial direction thereof. Specifically, the metal wire 21 making up the axial expansion-controlling member 20 is provided in a freely expansible state within a range controlled in the axial direction of the metal coil 11, whereby the metal wire 21 is pulled in the axial direction of the metal coil attending on the expansion of the metal coil 11 when the metal coil 11 is expanded in the axial direction of the coil, so that the metal wire 21 can be moved up to a state tensed to the metal coil 11.

Accordingly, the metal wire 21 does not inhibit the flexibility of the metal coil 11 itself even when both ends of the metal wire 21 are not fixed to the metal wire 11, and the intracorporeally indwelling device 10 can be made up as that having high flexibility.

In addition, in such a placement operation as described above, in order to arrange the intracorporeally indwelling device 10 at a proper position in an appointed site, it may be necessary in some cases to pull the intracorporeally indwelling device 10 arranged in a vital body once back within the catheter, thereby recovering it to conduct over the placement operation again. According to the intracorporeally indwelling device 10 of the present invention, however, the metal wire 21 is provided within the metal coil 11, and the metal wire 21 itself is made up of a material having high tensile break strength, whereby the expansion of the metal coil 11 in the axial direction of the coil can be controlled. Thus, upon conducting a replacement operation that the intracorporeally indwelling device is recovered to correct its position, for example, after it is arranged in a vital body once, the metal coil 11 is pulled within the catheter in a state that the metal coil 11 has been prevented by the stiffness of the metal wire 21 from being more expanded in the axial direction of the coil when the metal wire 21 is moved up to a state tensed to the metal coil 11, so that the replacement operation including the recovery of the intracorporeally indwelling device 10 can be surely performed, and the intracorporeally indwelling device 10 can be made up as that having high safety.

Further, by the construction (see Fig. 4) that only one end of the fiber element 31 making up the axial expansion-controlling member 30 is fixed to the metal coil 11, and the stopping member 25 is provided at the other end thereof, or the construction (see Fig. 5) that the fiber element 31 is not fixed to the metal coil 11, and the stopping members 25, 25 are provided at both ends thereof, the metal coil 11 is in a state that the degree of freedom to the fiber element 31 is high, so that the flexibility of the metal coil 11 is not inhibited by the fiber element 31, and the intracorporeally indwelling device 10 can be made up as that with high flexibility. Accordingly, high operating ability can be achieved upon conducting a placement operation, and the intracorporeally indwelling device 10 can be surely introduced and placed into an appointed site through a proper catheter. In addition, as a result that the expansion of the metal coil 11 in the axial direction thereof is controlled by the stopping member 25, upon conducting the replacement operation that the intracorporeally indwelling device is recovered to correct its position, for example, after the device is arranged in a vital body once through a proper catheter, the metal coil 11 is pulled into the catheter in a state that the metal coil 11 has been prevented by the stiffness of the fiber element 31 from being more expanded in the axial direction thereof when the metal coil 11 has been expanded within the controlled range, so that the replacement operation including the recovery of the intracorporeally indwelling device 10 can be surely performed, and the intracorporeally indwelling device 10 can be made up as that having high safety.

According to the intracorporeally indwelling device 10 of the present invention, the axial expansion-controlling member 20 is made up of a twisted wire 21A obtained by twisting a plurality of metal wires (see Fig. 3), whereby the tensile break strength of the axial expansion-controlling member in the axial direction of the coil can be made higher compared with that of a simple-wire structure, so that the diameter of each of the metal wires making up the twisted wire 21A can be made small. As a result, the flexibility of the metal wire itself becomes high, and the axial expansion-controlling member 20 can be surely made up as that having high flexibility though it has the expected strength.

Further, the axial expansion-controlling member 20 is made up of a tungsten wire material or a tungsten alloy wire material, whereby the diameter thereof can be made smaller compared with other metal wires having biocompatibility because tungsten or tungsten alloy itself has extremely high strength. Accordingly, the axial expansion-controlling member 20 can be surely made up as that having high strength and high flexibility in combination.

Although the preferred embodiments of the present invention have been described above, the present invention is not limited to the above-described embodiments, and such changes as described below may be added to the present invention.
(1) In the intracorporeally indwelling device 10 of the construction shown in Fig. 1, the axial expansion-controlling member 20 may be made up by spirally winding the metal wire to shape it to a coiled form or by shaping the metal wire to a zigzag or ribbon form.
   In this case, it is only necessary to set the coil diameter, coil length and other constitution of a coil to be formed by the metal wire in such a manner that the degree of expansion of the metal coil 11 in the axial direction thereof is controlled below a certain limit, and the intracorporeally indwelling device has predetermined flexibility.
(2) The coiled body is not limited to that made up of the metal coil, and it may be made up of a coil formed of, for example, a resin.

Further, the coiled body may be made up of that in which a fibrous material such as polyester is woven into the coiled body in the form of a loop, or that in which the surface of the coiled body is covered with a fibrous material woven in a cylindrical form. According to the coiled body of such a construction, the embolizing ability at an appointed site can be more improved.

### [Experimental Example]

Experimental Examples carried out for confirming the action and effect of the intracorporeally indwelling devices according to the present invention will hereinafter be described.

### <Production Example 1>

An axial expansion-controlling member composed of a tungsten wire having an element wire diameter of 20 µm and an overall length of 30.3 mm and formed in a state having a spiral structure was inserted into and arranged within a metal coil (coil diameter: 250 µm, coil length: 30 mm, the number of turns per mm: 20) formed by a platinum-tungsten alloy wire material having an element wire diameter of 50 µm, and at both ends of the metal coil, the outer surface of the both ends of the tungsten wire and the inner surface of the metal coil were fixed to each other with an adhesive, thereby producing such an intracorporeally indwelling device of a simple-wire structure as illustrated in Fig. 1, in which the tungsten wire was in a state freely expansible in an axial direction of the coil. This device is referred to as "Indwelling Device 1".

### <Production Example 2>

Such an intracorporeally indwelling device of a twisted-wire structure as illustrated in Fig. 3 was produced in the same manner as in Production Example 1 except that in Production Example 1, the axial expansion-controlling member was made up of a twisted wire formed by loosely twisting 3 tungsten wires each having an element wire diameter of 10 µm and an overall length of 30.6 mm. This device is referred to as "Indwelling Device 2".

The twisted wire is formed by twisting the tungsten wires together under a tension (twisting stress) of 500 N/mm², and the pitch of the twisted wire is 0.7 mm.

### <Production Example 3>

An axial expansion-controlling member made up of a fiber element having a diameter of 20 µm and a length of 160 mm and composed of polyvinyl alcohol (PVA) was inserted into a metal coil (coil diameter: 300 µm, coil length: 150 mm, the number of turns per mm: 33) formed by a tungsten wire having an element wire diameter of 30 µm, the outer surface of the fiber element and the inner surface of the metal coil were fixed to each other at a distal end of the metal coil with an adhesive, and a stopping member was fixed to the fiber element at a position located outside by 10 mm in an axial direction of the coil from the proximal edge of the metal coil, thereby producing an intracorporeally indwelling device of such a structure as illustrated in Fig. 4. This device is referred to as "Indwelling Device 3".

### <Production Example 4>

An intracorporeally indwelling device of such a structure as illustrated in Fig. 5 was produced in the same manner as in Production Example 3 except that in Production Example 3, the fiber element was arranged without being fixed to the metal coil, and stopping members were respectively provided on the fiber element at positions located outside by 5 mm in an axial direction of the coil from both edges of the metal coil. This device is referred to as "Indwelling Device 4".

### <Production Examples 5 to 7>

Such intracorporeally indwelling devices of a simple-wire structure as illustrated in Fig. 1 were produced in the same manner as in Production Example 1 except that in Production Example 1, the material making up the axial expansion-controlling member was changed in accordance with the following Table 1. These devices are referred to as "Indwelling Device 5" to "Indwelling Device 7". In Table 1, the numeral shown on a lower line in Material indicates tensile break strength inherent in the constituent material as recited in claim 1.

### <Comparative Production Example 1>

An intracorporeally indwelling device of a simple-wire structure was produced in the same manner as in Production Example 1 except that in Production Example 1, the axial expansion-controlling member was made up of a simple tungsten wire having an element wire diameter of 30 µm and an overall length of 30.3 mm. This device is referred to as "Comparative Indwelling Device 1".

### <Referential Production Example 1>

An intracorporeally indwelling device of a simple-wire structure was produced in the same manner as in Production Example 1 except that in Production Example 1, the axial expansion-controlling member was made up of a tungsten wire having the same overall length (30 mm) as the coil length of the metal coil in the ordinary state, and this tungsten wire was fixed to the metal coil in a tensed state. This device is referred to as "Referential Indwelling Device 1".

**[Table 1]**

| | Axial expansion-controlling member | | |
|---|---|---|---|
| | Material (break strength; N/mm²) | Structure Structure | Element wire diameter (µm) |
| Prod. Ex. 1 | W (4200) | Simple-wire; expansible | 20 |
| Prod. Ex. 2 | W (4200) | Twisted wire; expansible | 10 x 3 |
| Prod. Ex. 3 | PVA (700) | Fiber element; movable | 20 |
| Prod. Ex. 4 | PVA (700) | Fiber element; movable | 20 |
| Prod. Ex. 5 | Pt/W8 (1500) | Simple-wire; expansible | 20 |
| Prod. Ex. 6 | Ti-Ni (1200) | Simple-wire; expansible | 20 |
| Prod. Ex. 7 | SUS316 (1680) | Simple-wire; expansible | 25 |
| Comp. prod. Ex. 1 | W (4200) | Simple-wire; expansible | 30 |
| Ref. Prod. Ex. 1 | W (4200) | Simple-wire; tensed state | 20 |

With respect to Indwelling Devices 1 to 7, Comparative Indwelling Device 1 and Referential Indwelling Device 1 produced in the above-described manner, (1) tensile break strength (tensile break strength in those of the specified structures) of the respective axial expansion-controlling members in the axial direction of the coil and (2) flexibility of the whole of the indwelling devices were measured. The results are shown together with the maximum elongation rates of the metal coils in Table 2.

The tensile break strength of the axial expansion-controlling member is a value obtained by using a tension and compression tester "Strograph E-L" (manufactured by Toyo Seiki Seisaku-Sho, Ltd.) and chucking both ends of the metal coil in the indwelling device under an ordinary-temperature environment to conduct a tension test under conditions of a measuring load range of 2.5 NFS and a pulling rate of 100 mm/min.

The flexibility of the indwelling device itself is a value measured in accordance with the above-described method.

**[Table 2]**

| | Elongation rate of metal coil [%] | Tensile break strength of axial expansion-controlling member [N] | Flexibility of indwelling device as a whole [mN/mm] |
|---|---|---|---|
| Indwelling Device 1 | 1 | 1.32 | 20 |
| Indwelling Device 2 | 2 | 0.99 | 15 |
| Indwelling Device 3 | 3 | 0.22 | 8 |
| Indwelling Device 4 | 3 | 0.22 | 6 |
| Indwelling Device 5 | 2 | 0.47 | 16 |
| Indwelling Device 6 | 2 | 0.38 | 16 |
| Indwelling Device 7 | 2 | 0.82 | 18 |
| Comp. Indwelling Device 1 | 1 | 3.00 | 45 |
| Ref. Indwelling Device 1 | 0 | 1.32 | 40 |

From the results described above, it was confirmed that according to the intracorporeally indwelling devices (Indwelling Devices 1 to 7) related to the present invention, the axial expansion-controlling members can be made up as those having sufficiently high tensile break strength, whereby expansion of the metal coil in the axial direction thereof beyond a certain limit can be controlled, and moreover the intracorporeally indwelling devices can be made up as those having high flexibility, whereby high operating ability and high safety can be achieved upon conducting a placement operation.

Since the metal coil (coiled body) is deformed within an elastic region so far as the elongation rate of the metal coil does not exceed a yielding point, the metal coil can be restored to its original form when the load applied to the metal coil is released, so that the properties of the metal coil are not impaired from the viewpoint of practical use. When a replacement operation is performed, however, it is desirable that the elongation rate of the metal coil be as low as possible. According to Indwelling Devices 1 to 7 related to the present invention, the elongation rate of the metal coil can be controlled to about 1 to 3%. It is thus considered that the replacement operation can be surely performed.

On the other hand, in Comparative Indwelling Device 1, the diameter of the axial expansion-controlling member is relatively great, so that the flexibility of the indwelling device itself becomes low. It is thus considered that difficulty may be encountered upon surely conducting a placement operation of the intracorporeally indwelling device in some cases.

In Referential Indwelling Device 1, the axial expansion-controlling member has sufficiently high tensile break strength and is provided within the metal coil in a tensed state, so that the metal coil is substantially not expanded in the axial direction thereof. However, it is considered that the flexibility of the indwelling device itself is low, and so difficulty may be encountered upon surely conducting a placement operation of the intracorporeally indwelling device in some cases.

## Claims

1. An intracorporeally indwelling device for embolization, comprising a flexible coiled body and an axial extension-controlling member having tensile break strength greater than the minimum stress required for the coiled body to be permanently deformed by the axial extension thereof and provided in the interior of the coiled body so as to extend in an axial direction of the coiled body,
wherein the axial extension-controlling member is made up of a wire material having tensile break strength of at least 700 N/mm² in the axial direction of the coiled body and a diameter of at most 25 µm.

2. The intracorporeally indwelling device for embolization according to claim 1, wherein the wire material making up the axial expansion-controlling member is composed of a metal.

3. The intracorporeally indwelling device for embolization according to claim 2, wherein the metallic wire material making up the axial expansion-controlling member is tungsten.

4. The intracorporeally indwelling device for embolization according to claim 2, wherein the metallic wire material making up the axial expansion-controlling member is an alloy containing tungsten.

5. The intracorporeally indwelling device for embolization according to claim 1, wherein the wire material making up the axial expansion-controlling member is composed of a polymeric material.

6. The intracorporeally indwelling device for embolization according to any one of claims 1 to 5, wherein the axial extension-controlling member is made up of a twisted wire obtained by twisting a plurality of wire materials loosely.

7. The intracorporeally indwelling device for embolization according to any one of claims 1 to 6, wherein the coiled body is shaped to a secondary form.
